# EUROPEAN PATENT APPLICATION

(11) **EP 4 834 197 A1**
(43) Date of publication of application: **04.11.2026**
(21) Application number: 23962794.6
(22) Date of filing: 29.12.2023
(51) Int. Cl.: A61B 1/005, A61B 1/00

(54) **BENDING ADJUSTMENT DRIVING APPARATUS AND ENDOSCOPE**

(71) Applicant: Macrolux Medical Technology Co., Ltd., Shenzhen, Guangdong 518132 (CN)
(72) Inventor: ZHANG, Yong, Shenzhen, Guangdong 518132 (CN); WANG, Fei, Shenzhen, Guangdong 518132 (CN); WANG, Jin, Shenzhen, Guangdong 518132 (CN); WU, Wenhao, Shenzhen, Guangdong 518132 (CN)
(74) Representative: Viering, Jentschura & Partner mbB Patent- und Rechtsanwälte
(86) International application number: PCT/CN2023/143293
(87) International publication number: WO 2025/138119

(57) **Abstract**

The present invention relates to an articulation drive mechanism, particularly to the bending control of an endoscope. The articulation drive mechanism includes: a mounting base; a drive wheel, rotatably mounted to the mounting base; and at least one pair of pull wires, two pull wires of each pair arranged on opposite sides of a bending section to drive the bending section to pivot in two opposite directions ; the drive wheel is provided with at least two wire-winding regions including at least one first wire-winding region and at least one second wire-winding region, the first wire-winding region and the second wire-winding region are disposed at radially opposed sides of the drive wheel, and a winding radius of the at least one first wire-winding region differs from that of the at least one second wire-winding region. The invention is to address how to make the articulation drive mechanism meet the bending control requirements of the adjustable articulation section.

## Description

### TECHNICAL FIELD

The present application relates to an articulation drive mechanism, and more particularly, to bending control in an endoscope.

### BACKGROUND OF THE INVENTION

Endoscopes are inspection devices that integrate traditional optics, ergonomics, precision mechanics, modern electronics, mathematics, software, and other related disciplines. With the rapid advancement of science, technology, and medical techniques in recent years, endoscopes have been widely adopted for minimally invasive or non-invasive medical examinations or treatments. Generally, an endoscope includes a handle assembly and an insertion tube assembly. The insertion tube assembly may include an imaging module and is configured to be inserted into a target area to enable imaging thereof. A proximal end of the insertion tube assembly is coupled to a distal end of the handle assembly. The handle assembly is operable to drive the insertion tube assembly to move and/or rotate.

Particularly for medical flexible endoscopes, the distal end of the insertion tube assembly includes an adjustable articulation section (commonly known as bending section) capable of bending. As is known to those skilled in the art, the bending section generally includes multiple sequentially arranged bending segments. Hinge portions are provided between adjacent bending segments, enabling them to pivot relative to each other about an articulation axis defined by the hinge portions, thereby achieving bending of the bending section. During examination or treatment, a doctor can take advantage of the bendable nature of the endoscope's distal end by operating the articulation drive mechanism at the handle to articulate the bending section. This allows the flexible endoscope to enter the body in a minimally invasive manner through natural body orifices, thereby enabling omnidirectional visualization of the lesion tissue.

The articulation of the adjustable articulation section is controlled by pull wires. The pull wires are typically arranged in pairs, with each pair extending parallel to the axis of the insertion tube assembly and positioned on diametrically opposed sides of the articulation axis of the bending segments. When the articulation drive mechanism on the handle assembly applies tension to the pull wire on one side while slackening the pull wire on the opposite side, the adjustable articulation section bends toward the tensioned wire.

However, variations in bending control demands across different insertion tube assemblies require the articulation drive mechanism on the handle assembly to accommodate these differentiated bending control demands.

### SUMMARY OF THE INVENTION

### Technical Problem

The present invention primarily addresses the technical challenge of enabling the articulation drive mechanism to adaptively accommodate the bending control demands of adjustable articulation sections.

### Technical Solutions

According to a first aspect, an articulation drive mechanism is provided in some embodiments of the present invention.

The articulation drive mechanism may include:
a mounting base;
a drive wheel, rotatably mounted to the mounting base; and
at least one pair of pull wires, each pair arranged on opposite sides of a bending section to drive the bending section to pivot in two opposite directions;
the drive wheel is provided with at least two wire-winding regions including at least one first wire-winding region and at least one second wire-winding region;
the first wire-winding region and the second wire-winding region are disposed at radially opposed sides of the drive wheel; and
the at least one first wire-winding region has a different winding radius from that of the at least one second wire-winding region.

In some embodiments, the at least two wire-winding regions are arranged in layers along a rotational axis of the drive wheel.

In some embodiments, grooves are provided in a circumferential surface of the drive wheel, and the wire-winding regions are defined by respective groove.

In some embodiments, the drive wheel includes an anti-detachment protrusion disposed on a sidewall of the groove and positioned at a rim of the groove, the anti-detachment protrusion being configured to prevent disengagement of the pull wires from the groove in a radial direction of the drive wheel.

In some embodiments, the anti-detachment protrusion is disposed on one sidewall of a groove, a clearance opening is formed on the opposite sidewall of the groove, at least a portion of the anti-detachment protrusion protrudes into the clearance opening, and a gap is provided between the anti-detachment protrusion and an inner sidewall of the clearance opening.

In some embodiments, a bottom wall of the groove and an inner sidewall of the clearance opening are disposed on the same surface.

In some embodiments, the drive wheel includes an arc segment and a notch portion, the wire-winding regions are disposed on the arc segment, at least a portion of an end surface of the arc segment is exposed within the notch portion, and the pull wires of the articulation drive mechanism are wound onto the arc segment via the notch portion.

In some embodiments, the drive wheel has exactly one arc segment and exactly one notch portion, wherein the arc segment is a major arc, and the notch portion is disposed between the two ends of the major arc.

In some embodiments, the drive wheel is circular, and the notch portion forms a reduced-thickness region on the drive wheel. The reduced-thickness region includes a sloped surface portion whose thickness progressively decreases from a side proximal to the rotation axis of the drive wheel toward a radially outer side of the drive wheel. The sloped surface portion forms a smooth curved surface for supporting the pull wires.

In some embodiments, a fixing groove is disposed on an end surface of the drive wheel, an end of each pull wire is secured to a pull block; the pull block is received in the fixing groove, and a wall of the fixing groove is provided with a passage configured to lead out the pull wires.

According to a second aspect, an endoscope is provided in some embodiments of the present invention.

The endoscope may include:
an insertion tube assembly, wherein the insertion tube assembly includes an adjustable articulation section, the adjustable articulation section includes sequentially hinged bending segments, hinge portions are disposed between each pair of adjacent bending segments, the hinge portions define a pivot axis configured for relative deflection between the adjacent bending segments, and the hinge portions between each pair of adjacent bending segments are disposed at two radially opposite sides of the adjustable articulation section; and
a handle assembly, wherein the handle assembly is connected to a proximal end of the insertion tube assembly, and the handle assembly incorporates the articulation drive mechanism according to any one of the embodiments mentioned above.

In some embodiments, an extension direction of an axis of the bending segments is a first direction, the pivot axis extends in a second direction, each bending segment has a third direction perpendicular to both the first direction and the second direction, passages for the pull wires include a first pull-wire channel and a second pull-wire channel respectively disposed on two sides of the pivot axis along the third direction, and at least one of the first pull-wire channel and the second pull-wire channel is offset along the second direction on the bending segments.

In some embodiments, a perpendicular line from the first pull-wire channel to the pivot axis passes through the axis of the bending segments, the second pull-wire channel is offset, at least in the second direction, relative to a symmetrical position of the first pull-wire channel, the symmetrical position of the first pull-wire channel is a mirror image position of the first pull-wire channel with respect to a symmetry plane containing both the axis of the bending segments and the pivot axis.

In some embodiments, the bending segment is provided with: one working channel forming a channel distribution region; or at least two working channels, wherein portions of the bending segment between the working channels and the working channels collectively form a channel distribution region; the first pull-wire channel is disposed externally to the channel distribution region in the third direction, and the second pull-wire channel is disposed externally to the channel distribution region in the second direction.

### Beneficial Effects

The beneficial effects of the present invention are as follows:
By providing different wire-winding regions on the drive wheel, different wire-winding radii are achieved. Paired pull wires may be wound on respective wire-winding regions to attain different wire-winding radii. When the drive wheel rotates, the pull wires undergo differential length variations due to their different wire-winding radii, thereby satisfying corresponding bending control requirements for the adjustable articulation sections. This configuration eliminates the need for separate articulation drive mechanisms for each paired pull wire, yielding a simplified structure, convenient operation, and reduced costs.

Further, when the drive wheel incorporates multiple wire-winding regions, a single articulation drive mechanism can accommodate diverse bending angles across different adjustable articulation sections. This enables different endoscope products to share a common driving device while facilitating assembly, ensuring ease of implementation, and effectively reducing material variety along with product costs.

### BRIEF DESCRIPTION OF THE DRAWINGS

FIG. 1 is an exploded perspective view of a handle assembly in an embodiment of an endoscope.
FIG. 2 is a front view of the articulation drive mechanism in FIG. 1.
FIG. 3 is a cross-sectional view of the drive wheel's mounting structure in FIG. 1.
FIG. 4 is a first perspective view of the drive wheel in FIG. 1.
FIG. 5 is a second perspective view of the drive wheel in FIG. 1.
FIG. 6 is a schematic diagram of the pull wires in FIG. 3.
FIG. 7 is a front view of the drive wheel in FIG. 1.
FIG. 8 is a cross-sectional view taken along line A-A of the drive wheel in FIG. 1.
FIG. 9 is a cross-sectional view taken along line B-B of the drive wheel in FIG. 1.
FIG. 10 is a perspective view of a bending section in an embodiment of an endoscope.
FIG. 11 is an enlarged partial view of FIG. 10.
FIG. 12 is a longitudinal cross-sectional view of FIG. 10.
FIG. 13 is a right-side view of FIG. 12.
FIG. 14 is an enlarged partial view of FIG. 12.
FIG. 15 is a perspective view of a bending section in another embodiment of the present invention.
FIG. 16 is a schematic showing the layout of pull-wire channels on the bending segment in FIG. 15.

### Reference Numerals List:

- 110: lower housing;
- 120: upper housing;
- 200: drive wheel;
- 210: protruding column;
- 211: square aperture;
- 221: fixing groove;
- 222: passage;
- 231: first wire-winding region;
- 232: second wire-winding region;
- 233: anti-detachment protrusion;
- 234: clearance opening;
- 241: arc segment;
- 242: notch portion;
- 250: zero-position locating notch;
- 300: power input member;
- 310: drive shaft;
- 311: square terminal;
- 312: operating rod;
- 313: screw;
- 410: distal connection part;
- 420: proximal connection part;
- 430: bending segment;
- 431: working channel;
- 432: first pull-wire channel;
- 433: second pull-wire channel;
- 434: connection wire channel;
- 435: recess;
- 440: hinge portion;
- 441: pivot axis;
- 450: connection wire;
- 460: pull wire;
- 461: protective sleeve;
- 462: pull block.

### DETAILED DESCRIPTION

Specific embodiments of the present invention will now be described in detail with reference to the accompanying drawings. Similar or related components in different embodiments are labeled with associated reference numerals. The following embodiments include detailed descriptions to facilitate understanding of the present invention. However, those skilled in the art will readily recognize that certain features may be omitted under specific circumstances or substituted by other components, materials, or methods. In some instances, certain operations related to the present invention are not explicitly described or illustrated herein. This intentional exclusion is intentional to avoid obscuring the core technical solutions of the present invention. For those skilled in the art, a complete understanding of these operations can be attained through the descriptions provided in this specification and general technical knowledge in the art.

Additionally, the features, operations, or characteristics described in the specification may be combined in any suitable manner to form various embodiments. Similarly, steps or actions in the method descriptions may be reordered or modified in ways that would be obvious to those skilled in the art. Therefore, the sequences presented in the specification and drawings are intended solely to clarify the description of specific embodiments and do not imply mandatory orderings, unless explicitly stated that a particular sequence is required.

The numerical designations assigned to components in this specification, such as 'first,' 'second,' or similar ordinal terms, serve solely to distinguish described objects and carry no inherent sequential or technical implications. Furthermore, the terms 'connected' and 'coupled' as used herein encompass both direct and indirect connection (coupling), unless explicitly stated otherwise.

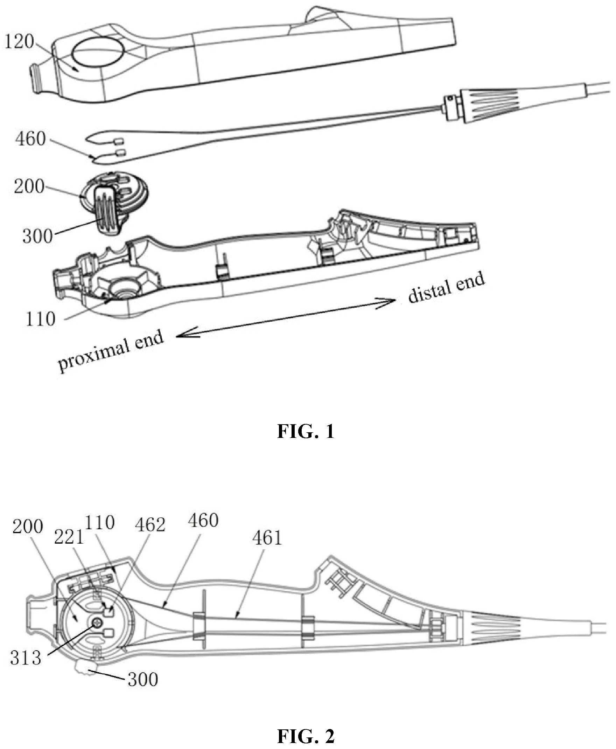

The terms "proximal" and "distal" as used herein are conventional in the medical field. With respect to the instrument to be operated, the proximal end refers to the end closer to the operator, while the distal end refers to the end farther away from the operator. In FIG. 1, the proximal and distal ends are marked with arrows.

In the present invention, the drive wheel 200 of the articulation drive mechanism is provided with wire-winding regions having different winding radii. Paired pull wires used to respectively drive a bending section to articulate in two opposite directions can be wound on different wire-winding regions of the drive wheel 200, ensuring different winding radii for the paired pull wires. When the drive wheel 200 rotates by the same angle, different pull wires can exhibit different changes in length, thereby accommodating different bending adjustment requirements.

An embodiment of an articulation drive mechanism in the present invention is disclosed as follows:
Referring to FIG. 1, in an embodiment, an articulation drive mechanism is provided on a handle assembly of an endoscope. The handle assembly includes a lower housing 110, an upper housing 120, a drive wheel 200, a power input member 300, and pull wires 460.

The lower housing 110 and the upper housing 120 can be fixed together, defining an installation cavity for installing corresponding components. The fixation methods of the lower housing 110 and the upper housing 120 may be ultrasonic welding, snap connection, fastener connection, bonding, etc. Meanwhile, the lower housing 110 defines the mounting base of the articulation drive mechanism and can install the drive wheel 200, the power input member 300, and the pull wires 460.

The drive wheel 200 is rotatably mounted on the mounting base and configured to be rotated by a manual operator. When the drive wheel 200 rotates, it can wind one pull wire 460 while simultaneously releasing the other, thereby changing the effective length of the pull wires 460 between the bending section and the drive wheel 200 of the endoscope to achieve the bending adjustment of the endoscope. Specifically, the pull wires 460 may be arranged in pairs, with paired pull wires 460 being respectively arranged on different sides of the bending section; and when one of the two pull wires 460 in a pair is wound and tightened, the other is released and relaxed, thereby articulating the bending section in opposite directions.

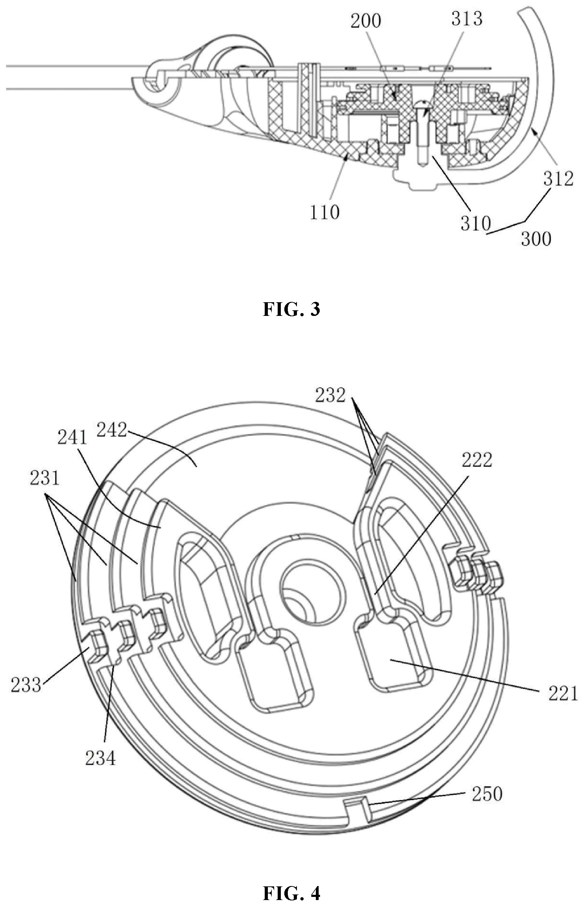

In an embodiment, a transmission structure is disposed at an axial side of the drive wheel 200 for coupling with the power input member 300 to enable forward and reverse rotation of the drive wheel 200. In a specific embodiment (FIGS. 3, 5 and 7), the drive wheel 200 includes a protruding column 210 adjacent to the lower housing 110, the protruding column 210 having a square aperture 211. The power input member 300 is configured as a toggle lever including an operating rod 312 and a drive shaft 310 connected to one end of the operating rod 312. The drive shaft 310 includes a square terminal 311 configured to be received in the square aperture 211 for torque transmission. Further, as illustrated in FIG. 3, the lower housing 110 is formed with a mounting hole through which the drive shaft 310 extends. Said drive shaft 310 is secured to the drive wheel 200 inside the lower housing 110 via a screw 313, such that the drive wheel 200 is co-located with it on the lower housing 110, and rotatable relative to the lower housing 110.

In other embodiments, the transmission structure may also be replaced by any other form capable of achieving power transmission, such as providing a square terminal on the drive wheel 200 and a square aperture on the power input member 300, or replacing the square aperture 211 and the square terminal 311 with hexagonal, triangular or other polygonal shapes, or using a cylinder with a flat outer surface. Additionally, in the above embodiments, the drive wheel 200 is manually driven by an operator. In some cases, the drive wheel 200 may also be driven electrically. Moreover, the drive wheel 200 and the toggle lever may be connected in other ways, such as interference fit, snap fit, bonding, and so on.

The proximal end of each pull wire 460 is fixed on the drive wheel 200. In an embodiment, a fixing groove 221 is provided on an end surface of the drive wheel 200; and the end of each pull wire 460 is fixed with a pull block 462, which is received in the fixing groove 221. The groove wall of the fixing groove 221 has a passage 222 for the pull wire 460 to pass through, thereby achieving the fixation of the proximal end of the pull wire 460 to the drive wheel 200. To prevent the pull block 462 from coming out of the fixing groove 221, in an embodiment, adhesive liquid may be injected into the fixing groove 221, and after the adhesive liquid solidifies, the pull block 462 is bonded to the drive wheel 200. Of course, in other embodiments, the pull wire 460 may also be fixedly connected to the drive wheel 200 in other ways, such as by clamping with a screw. By adjusting the installation position of the pull block 462 on the pull wire 460, it may be ensured that the pull wire 460 is tensioned on the wire-winding region of the drive wheel 200 after the pull block 462 is installed on the drive wheel 200. The fixation method of the pull wire 460 and the pull block 462 is not limited; for example, it may be fixed by interference fit, clamping, bonding, or securing with a set screw. Additionally, in other embodiments, the fixing grooves 221 corresponding to the two pull wires 460 may also be adjusted in position, for example, located in the notch portion, and the fixing grooves 221 may be combined into a larger groove structure capable of simultaneously installing two pull blocks 462.

To ensure the smooth movement of the pull wires 460, in an embodiment, after the pull wires 460 are wound onto the drive wheel 200, the wires pass through a protective sleeve 461. The pull wires 460 may slide inside the protective sleeve 461, and the lower housing 110 has a positioning structure for fixing the protective sleeve 461, such as a snap-fit groove.

The power input member 300 of the articulation drive mechanism may be actuated by an operator's fingers. When the power input member 300 rotates, it drives the drive wheel 200 inside to rotate. As the drive wheel 200 rotates, it causes the pull wires 460 installed on it to change in length, thereby driving the bending section to bend. To facilitate the operation of medical staff and reduce the force required to operate the toggle lever, the maximum operable angle of the power input member 300 in the articulation drive mechanism of the bending section is generally selected to be in line with ergonomics. The operable angles of the levers of endoscope products with different maximum bending angles are basically the same. Under the premise that the operable angle of the toggle lever is fixed, the maximum bending angle of the bending section may be determined by the winding radius of the drive wheel 200. The winding radius is the radial distance from the center of the drive wheel 200 to the position on its outer periphery where the pull wire 460 is wound. Different winding radii result in different maximum bending angles of the bending section. The present invention provides a drive wheel 200 designed with multiple winding radii, allowing different maximum bending angles of the bending section to be achieved by selecting different winding radii.

A key consideration for designing multiple winding radii on the drive wheel 200 is the balance between reliability and cost of the bending section - a critical component in endoscopic devices. Currently, bending sections are generally categorized into two types: metal-based and non-metal-based. For metal bending sections: each bending segment is fabricated through blanking, cutting, and slotting processes. These steps demand high precision, leading to low productivity and high per-segment costs. For non-metal (primarily plastic) bending sections: integrated molding via injection achieves high efficiency and low cost. However, injection molding requires custom tooling, incurring substantial mold fabrication costs and extended lead time. Furthermore, bending angle requirements vary across endoscope models. Producing dedicated bending sections for each angle variant, whether metal or plastic, incurs prohibitively high costs. The present invention addresses this by redesigning the articulation drive mechanism: multiple winding radii on the drive wheel 200 enable a single bending section to achieve diverse bending angles.

Specifically, the drive wheel 200 is provided with at least two wire-winding regions, namely the first wire-winding region 231 and the second wire-winding region 232. The first wire-winding region 231 and the second wire-winding region 232 are positioned at radially distinct locations on different sides of the drive wheel 200 in the radial direction, and have different winding radii. In a specific embodiment, to prevent the pull wire 460 from detaching from the drive wheel 200 and to enhance operational reliability, the drive wheel 200 has grooves on its outer peripheral surface. Each wire-winding region is defined by a respective groove.

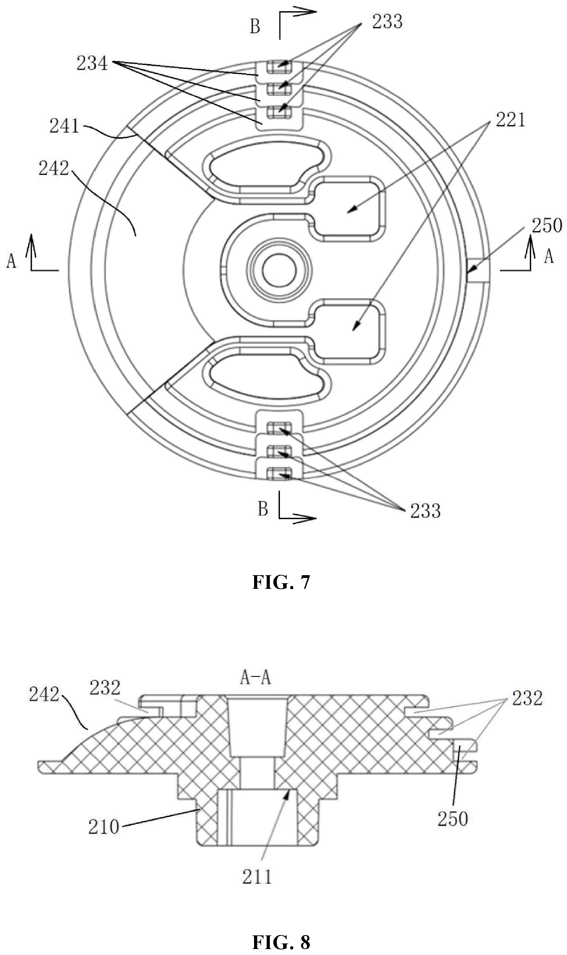

In an embodiment, to accommodate diverse bending requirements, at least two wire-winding regions are axially layered along the rotational axis of the drive wheel 200, forming a stepped configuration. In a specific embodiment, as shown in FIGS. 4, 7 to 9, the drive wheel 200 includes three first wire-winding regions 231 and three second wire-winding regions 232. With reference to FIG. 7, the three first wire-winding regions 231 are situated on the lower side of the drive wheel 200, and the three second wire-winding regions 232 are located on the upper side. From the side of the drive wheel 200 featuring the fixing groove 221 towards the opposite side, the winding radii of the three first wire-winding regions 231 increase successively, and those of the three second wire-winding regions 232 increase progressively. Moreover, each first wire-winding region 231 circumferentially adjoins a corresponding second wire-winding region 232 on the drive wheel 200, thereby combining to form a continuous arc. This structure facilitates the manufacturing of the drive wheel 200 and ensures the winding of the pull wire 460 even when the drive wheel 200 rotates at a large angle, enabling a greater traction stroke. Notably, the circumferentially adjoining first wire-winding region 231 and the second wire-winding region 232 may be considered as having an overlapping segment that is shared by the first wire-winding region 231 and the second wire-winding region 232.

It should be noted that in some other embodiments, both the number of the first wire-winding regions 231 and the second wire-winding regions 232 can be increased or decreased, and the number of the first wire-winding regions 231 may be different from that of the second wire-winding regions 232. Additionally, the first wire-winding regions 231 and the three second wire-winding regions 232 occupying the same axial position on the drive wheel 200 may be separated from each other along the circumferential direction.

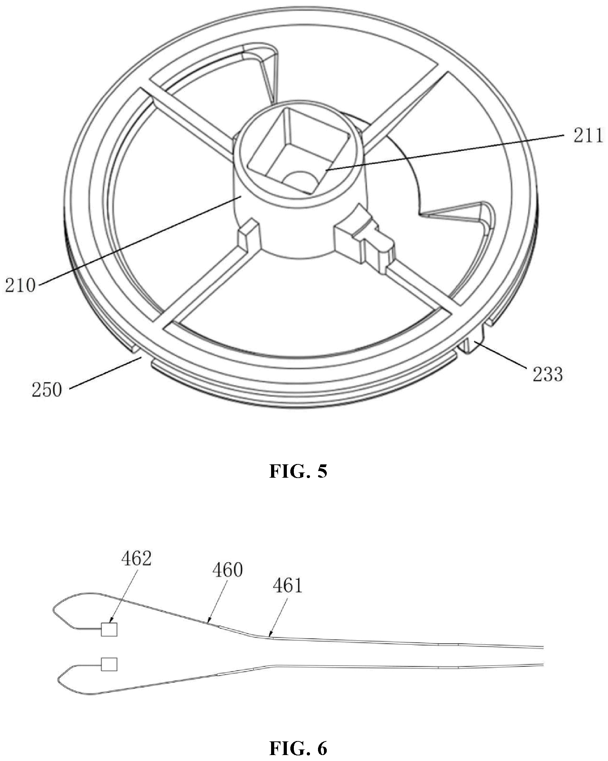

Referring to FIGS. 4, 5 and 7, in an embodiment, the drive wheel 200 is circular. The circumferential adjoining of the first and second wire-winding regions 231 and 232 collectively defines an arc segment 241 and a notch portion 242 on the drive wheel 200. The wire-winding regions are disposed on the arc segment 241, and at least a portion of the end surface of the arc segment 241 is exposed in the notch portion 242. The pull wire 460 of the articulation drive mechanism is threaded through the notch portion 242 and wound onto the arc segment 241. The fixed groove 221 on an end surface of the drive wheel 200 and the notch portion 242 are located on opposite sides of the drive wheel 200. A passage 222 provided in the sidewall of the fixed groove 221 proximate to the notch portion 242 permits the pull wire 460 to extend through it to the notch portion 242 and subsequently enter the wire-winding region via the notch portion 242, facilitating the assembly of the pull wire 460 onto the drive wheel 200. The initial state of the pull wire 460 when it is assembled onto the drive wheel 200 is shown in FIGS. 2 and 6.

According to the rotation angle of the drive wheel 200, in the illustrated embodiments, the arc segment 241 is a major arc, with the notch portion 242 located between the two ends of the major arc. However, in some other embodiments, depending on different rotation angles of the drive wheel 200, the arc segment 241 may be a minor arc, and a notch portion 242 may be disposed between the corresponding ends of both the first wire-winding region 231 and the second wire-winding region 232.

In an embodiment, the notch portion 242 defines a reduced-thickness region on the drive wheel 200, wherein the reduced-thickness region includes a sloped surface portion. The thickness of the sloped surface portion gradually reduces from the rotation-axis-proximal side toward the radial outer side of the drive wheel 200, forming a smooth curved surface configured to support the pull wires 460. This smooth curved surface is configured to protect the pull wires 460 by preventing formation of sharp edges on the drive wheel 200, thereby preventing wear or even breakage of the pull wires 460.

In some other embodiments, the notch portion 242 may extend completely through the drive wheel 200 along its axial direction, forming a fully open aperture. Additionally, each layer of the wire-winding region distributed along the axial direction of the drive wheel 200 may extend continuously around the entire circumference of the drive wheel 200. Where the wire-winding region extend circumferentially for a full circle, to enable the pull wires 460 with the terminus affixed on the end surface of the drive wheel 200 to enter the clamping groove serving as the wire-winding region, a wire-access passage 222 through which the traction wire 460 passes may extend from the fixed groove 221 to the corresponding wire-winding region.

To ensure that the pull wires 460 remain engaged with the drive wheel 200, the drive wheel 200 includes an anti-detachment protrusion 233. The anti-detachment protrusion 233 is disposed on the sidewall of a groove and located at the groove opening, and is configured to prevent the pull wires 460 from disengaging from the groove along the radial direction of the drive wheel 200. In some embodiments, the anti-detachment protrusion 233 may be disposed on one sidewall of the groove, and the other sidewall of the groove is provided with a clearance opening 234. At least a portion of the anti-detachment protrusion 233 extends into the clearance opening 234, and there is a gap between the anti-detachment protrusion 233 and the inner wall of the clearance opening 234. During assembly, the pull wires 460 may be inserted into the groove along the circumferential direction of the drive wheel 200. Further, the bottom wall of the groove and the inner wall of the clearance opening 234 are located on the same arcuate surface, which facilitates the forming of the drive wheel 200 and also makes it easier to observe the positioned status of the pull wires 460 in the groove. In some other embodiments, the bottom wall of the groove and the inner wall of the clearance opening 234 may also be located on different surfaces.

To accurately determine the initial position of the drive wheel 200, a zero-position locating notch 250 is also designed on the drive wheel 200. The zero-position locating notch 250 may be located at the symmetrical axis position of the first wire-winding region 231 and the second wire-winding region 232. In an embodiment, the zero-position locating notch 250 may be disposed on the sidewall of the groove that is the farthest from the fixing groove 221.

It should be noted that in the illustrated embodiments, the number of anti-detachment protrusions 233 on each wire-winding region is one, and they are disposed on both sides of the symmetry axis between the first wire-winding region 231 and the second wire-winding region 232. In some other embodiments, the anti-detachment protrusions 233 may be provided in greater numbers. Additionally, the anti-detachment protrusions 233 may also be disposed on both sidewalls of the groove.

The articulation drive mechanism mentioned above can accommodate different maximum bending angles for the same bending section, meeting the articulation requirements of different endoscopes. This enables multiple endoscope products to share one bending section drive mechanism, thereby avoiding the high costs associated with fabricating new bending sections.

When the bending section is assembled or combined with other components, interference between the pull wires 460 and other components often occurs. To solve this interference issue, the size of the bending section is typically increased. However, in the field of endoscopes, the size parameters of the part entering the human body are critical indicators. Their values directly impact the product's application scope, patient experience during examination or treatment, and the product's market competitiveness. Therefore, departing from the conventional structure of the bending section in the prior art, where the pull-wire channels are symmetrically distributed on both sides of the pivot axis, the present approach employs an asymmetric distribution of the first and second pull-wire channels relative to the pivot axis of the bending segments. This configuration frees up space in the corresponding region, thereby facilitating the reduction or avoidance of interference between the pull wires 460 and other component structures during the assembly of the bending segments with other components. It also avoids the need to increase the radial dimension to accommodate the pull wires 460, which is beneficial for controlling the radial dimension of the bending section.

Another consideration for designing multiple winding radii on the drive wheel 200 is as follows. For the bending section with asymmetrically arranged pull wires 460, the displacement of the pull wires 460 differs when the bending section bends in opposite directions, For the pull wires 460 on the side closer to the pivot axis of the bending section, its displacement during bending is relatively smaller and its traction force is relatively larger; for the pull wires 460 on the side farther from the pivot axis of the bending section, its displacement is relatively larger and its traction force is relatively smaller. If a traditional drive wheel 200 is used to drive such a bending section with asymmetric pull wire arrangement, problems such as inconsistent operational force required for bending the bending section in both directions or even jamming may occur (for example, if the winding radius of the drive wheel 200 is designed for the pull wires 460 requiring the smaller displacement, the pull wires 460 requiring the larger displacement will be unable to achieve sufficient travel, potentially leading to jamming). In the present invention, the first wire-winding region 231 and the second wire-winding region 232 with different winding radii are provided. The displacement and traction force differences of the pull wires 460 during bidirectional bending of the bending section, resulting from the asymmetric arrangement of the pull wires 460, can be balanced by designing wire-winding regions with corresponding winding radii on both sides of the drive wheel 200. Specifically, a smaller winding radius is used for the pull wire 460 on the side closer to the pivot axis of the bending section, and a larger winding radius is used for the pull wire 460 on the side farther from the pivot axis of the bending section. Moreover, the winding radii on both sides of the drive wheel 200 is directly related to the distances from the pull wires 460 on the respective sides of the bending section to the pivot axis. By designing appropriate winding radii, it is possible to achieve smooth bending of the bending section in both directions while maintaining a consistent operational feel.

It should be noted that for the bending section with asymmetrically arranged pull wires 460 mentioned above, the drive wheel 200 may be provided with one first wire-winding region 231 and one second wire-winding region 232. The first wire-winding region 231 and the second wire-winding region 232 can be located at the same position or at different positions along the axial direction of the drive wheel 200. Of course, for the drive wheel 200 provided with two or more first wire-winding regions 231 and second wire-winding regions 232, two wire-winding regions can be selected to drive the two pull wires 460 of the asymmetric bending section, provided that the bending requirements of the asymmetric bending section are met.

An embodiment of an endoscope in the present invention is disclosed as follows:
The endoscope includes an insertion tube assembly and a handle assembly. The insertion tube assembly includes a bending section. The bending section includes a plurality of sequentially hinged bending segments. Hinge portions are provided between each pair of adjacent bending segments. The hinge portions form a pivot axis allowing the two adjacent bending segments to pivot relative to each other. The hinge portions between adjacent bending segments are located on radially opposite sides of the bending section. The handle assembly is connected to the proximal end of the insertion tube assembly, and houses an articulation drive mechanism. The articulation drive mechanism is that of any one of the above embodiments.

The following will mainly introduce the structure of the above-mentioned asymmetric adjustable articulation section.

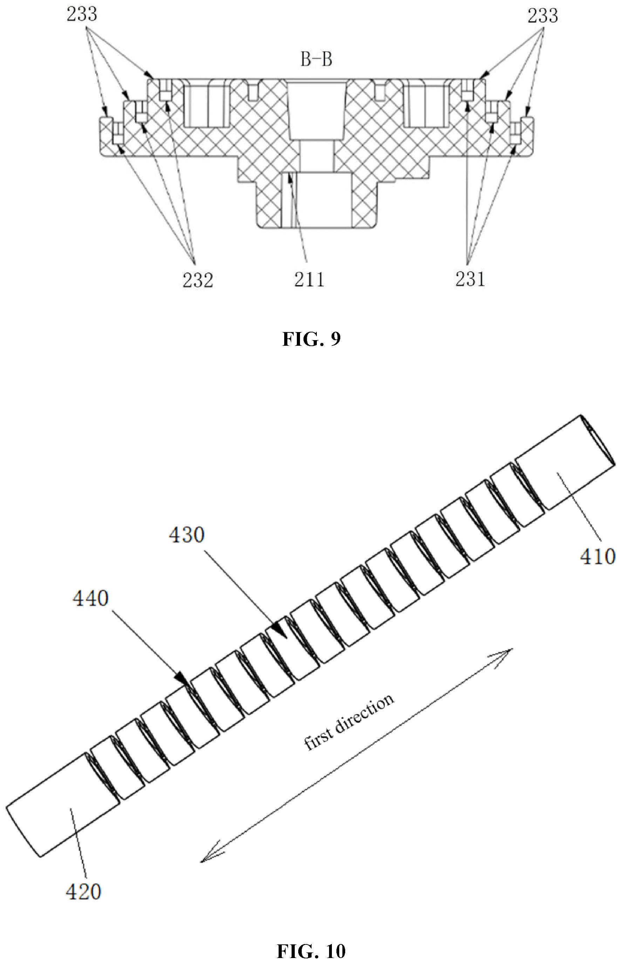

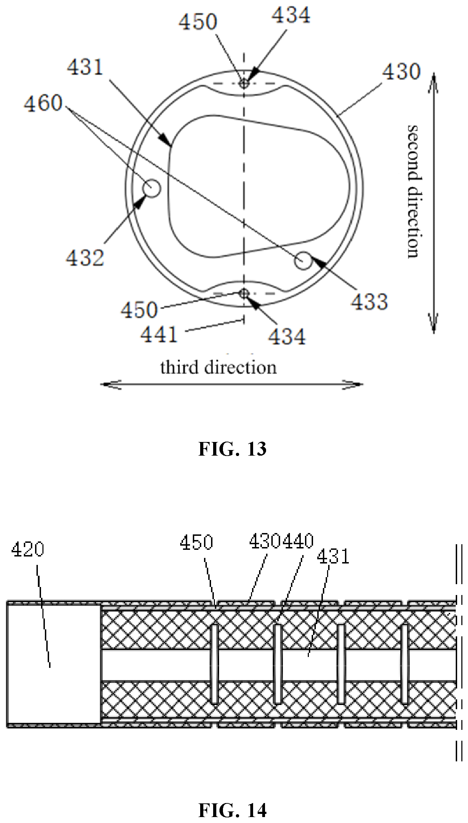

In an embodiment, as an important component of the insertion tube assembly of the endoscope, the adjustable articulation section (Please refer to FIGS. 10 and 13) includes a distal connection part 410, a proximal connection part 420, bending segments 430 disposed between the proximal connection part 420 and the distal connection part 410, hinge portions 440, and connection wires 450 extending through the bending segments 430 and the hinge portions 440.

Referring to FIGS. 10 to 13, the bending section is integrally formed by injection molding, and the material may be plastic or other materials suitable for injection molding. The connection wire 450 may be directly formed within the bending section as a pre-embedded insert during injection molding and may pass through each bending segment 430 and each hinge portion 440. The connection wires 450 may be made of metal or non-metal, with adequate strength and toughness, to enhance reliability and prevent failure of adjacent bending segments 430 during repeated deflection and bending. Of course, the connection wires 450 may be an optional structure. The connection wires 450 are located on the symmetrical centerline of the outer contour of the bending section, facilitating the establishment of a stable pivot axis 441 for each bending segment 430. In some other embodiments, the connection wires 450 may also be assembled with the bending segments 430 and the hinge portions 440 after formation of the bending segments 430 and the hinge portions 440.

The number of bending segments 430 may be increased or decreased as needed. These segments 430 are arranged along the axis of the bending section. During endoscope operation, the curvature of the axis of the bending section changes as the deflection of the bending segments 430. In an initial state, the bending section is generally straight with a linear axis. As the bending section bends, its axis correspondingly assumes a curved configuration.

The deflection and bending of each bending segment 430 is achieved through the hinge portions 440. The hinge portions 440 are connected between adjacent bending segments 430 and are located on both radially opposed sides of the bending section. Referring to FIG. 13, the hinge portions 440 form a pivot axis 441 for the relative deflection of adjacent bending segments 430. This pivot axis 441 may be considered as a line connecting two hinge portions 440 located on both radially opposed sides of the bending section. It should be noted that the pivot axis 441 may be a virtual axis or a solid axis. For example, for the bending section formed by one-piece injection molding in this embodiment, the pivot axis 441 of each bending segment is a virtual axis, while for the assembled bending section, the hinge portions 440 of each bending segment form a pivot axis 441 of solid structure. As an example, the axial end of a bending segment 430 may be provided with a clevis plate protruding from the end surface, and a rotation shaft perpendicular to the axis of the bending segment 430 is provided on the clevis plate, while the opposite axial end of the bending segments 430 may be provided with a rotation fit hole. The rotation shaft may be rotatably embedded in the rotation fit hole to achieve articulation.

In order to enable the passage of instruments and/or the routing of lines, the bending segments 430 are provided with at least one working channel 431. The working channel 431 extends along the axis of the bending section and is located in the central region of the cross-section of each bending segment 430. It should be noted that the number of working channels 431 may be configured as one or more (e.g., two or more). Any working channel 431 can accommodate only one type of instrument/line, or two or more different instruments/lines simultaneously. Additionally, the shape of the working channel 431 may be designed as needed and is not limited in this invention. When the bending segments 430 are provided one working channel 431, this single working channel 431 forms a channel distribution region, which corresponds to the edge contour of the working channel. In some other embodiments, the bending segments 430 may also be provided with at least two working channels. In such cases, the portions of the bending segments 420 located between the working channels and the working channels themselves together form a channel distribution region.

To achieve the bending control of the bending section, the bending segments 430 are provided with pull-wire channels, which are located outside the working channels 431 and also extend along the axis of the bending section. The pull-wire channels include a first pull-wire channel 432 and a second pull-wire channel 433. The first pull-wire channel 432 and the second pull-wire channel 433 are respectively arranged on different sides of the bending segments 430 along a direction perpendicular to the axis of the bending section. Each pull-wire channel is configured to accommodate a pull wire 460. When any one of the pull wires 460 is tightened, the bending section can bend specifically toward the side where the pull wire 460 is tightened. It should be understood by those skilled in the art that the pull-wire channels enable the movement of the pull wires 460 within them, and that the distal end of any pull wire 460 can be fixed to the distal end of the bending section.

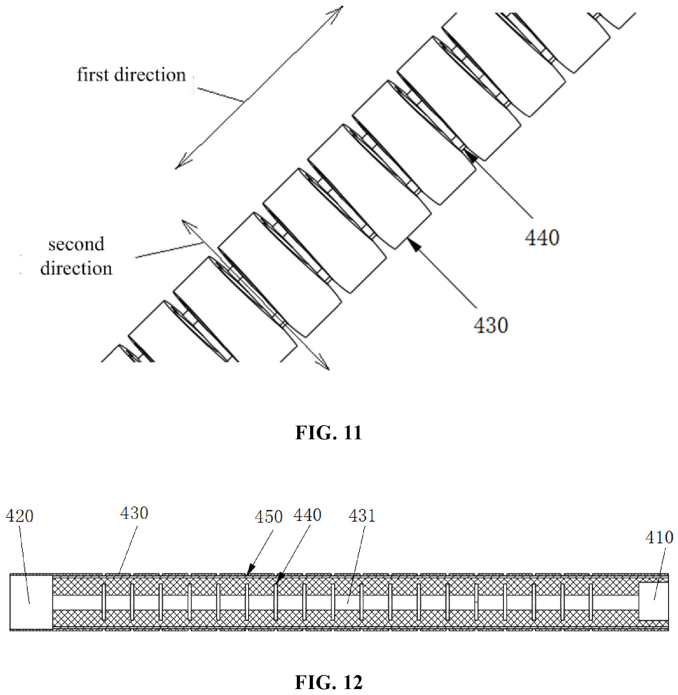

To facilitate a clearer explanation of the specific embodiments and technical solutions of the present invention, the following directional definitions are provided with reference to FIGS. 11 and 13: the extension direction of the axis of the bending segments 430 is defined as the first direction; the extension direction of the pivot axis 441 is defined as the second direction; and the direction simultaneously perpendicular to both the first and second directions with respect to the bending segments 430 is defined as the third direction. It should be understood that this description of orientation in the embodiments is made solely to more clearly describe the positional relationships among the various components and is not intended to limit the actual spatial orientation of the device according to the present invention.

In an embodiment, the outer cross-sectional contour of the bending section is circular, with the outer contour dimension in the second direction equal to the outer contour dimension in the third direction. However, in other embodiments, the outer cross-sectional contour of the bending section can also be of other shapes, such as elliptical, causing the outer contour dimension of the bending section in the second direction to differ from its outer contour dimension in the third direction.

Since the pull wires 460 are prone to interference during the assembly of the bending section with other components, addressing this issue by increasing the radial dimension of the bending section would compromise the scope of application of the endoscope and reduce patient comfort during examination and treatment. In the present invention, at least one of the first pull-wire channels 432 (located on one side of the pivot axis 441 in the third direction) and the second pull-wire channel 433 (located on the other side of the pivot axis 441 in the third direction) is offset along the second direction on the bending segments 430. This configuration avoids the need to increase the radial dimension of the bending section to create clearance, thereby enabling control over the radial dimension of the bending section. It should be understood by those skilled in the art that this "offset" refers to a deviation from the middle position of the bending segments 430 in the corresponding direction. The middle position of the bending segments 430 in the corresponding direction is located on the straight line, along the third direction, having the maximum outer contour dimension of the bending section.

In an embodiment, only the second pull wire 460 may be offset. Refer to FIG. 13, in an embodiment, the perpendicular line from the first pull-wire channel 432 to the pivot axis 441 passes through the axis of the bending segments 430, and the second pull-wire channel 433 is offset from the symmetrical position of the first pull-wire channel 432 at least in the extension direction of the pivot axis 441. The symmetrical position of the first pull-wire channel 432 is the mirror image position of the first pull-wire channel 432 with respect to the plane containing the axis of the bending segments 430 and the pivot axis 441.

In a specific embodiment, the distance from the first pull-wire channel 432 to the pivot axis 441 is greater than the distance from the second pull-wire channel 433 to the pivot axis 441. This arrangement allows for the formation of a larger clearance at the symmetrical position of the first pull-wire channel 432.

In a specific embodiment, as shown in FIG. 13, the working channel 431 has different dimensions along the second direction on both sides of the third direction, with the side away from the first pull-wire channel 432 having a smaller dimension. Correspondingly, the first pull-wire channel 432 and the working channel 431 are arranged along the third direction, that is, the first pull-wire channel 432 is positioned on the outer side of the channel distribution region in the third direction, while the second pull-wire channel 433 and the working channel 431 are arranged along the second direction, that is, the second pull-wire channel 433 is positioned on the outer side of the channel distribution region in the second direction. In other embodiments, where the second pull-wire channel 433 exhibits a minor positional deviation from symmetry relative to the first pull-wire channel 432, specially with a portion extending beyond the working channel 431 in the third direction, it can also be considered that the second pull-wire channel 433 and the working channel 431 are arranged along the third direction.

In an embodiment, referring to FIG. 13 along the third direction, the bending segments 430 defines a first side provided with the first pull-wire channel 432 and a second side provided with the second pull-wire channel 433. The wall thickness of the working channel 431 on the first side exceeds that on the second side, thereby enabling an enlarged cross-sectional workspace while allowing integration of the second pull-wire channel 433 without increasing the dimensions of the bending section.

To maximize the dimensions of the working channel 431, in an embodiment, the cross-sectional contour of the working channel 431 on the side away from the first pull-wire channel 432 is arc-shaped, enhancing spatial efficiency. Alternatively, in a specific embodiment, the cross-sectional contour of the working channel 431 on the side away from the first pull-wire channel 432 may adopt a linear configuration. Additionally, in some other embodiments, the working channel 431 may be symmetrically arranged along the pivot axis 441.

In the aforementioned embodiments, the bending section exhibits a circular peripheral profile in cross-sectional. In alternative embodiments, the cross-sectional outer contour of the bending section may assume non-circular geometries, such as an elliptical profile oriented with its major axis parallel to the third direction. This arrangement may achieve a smaller circumferential dimension.

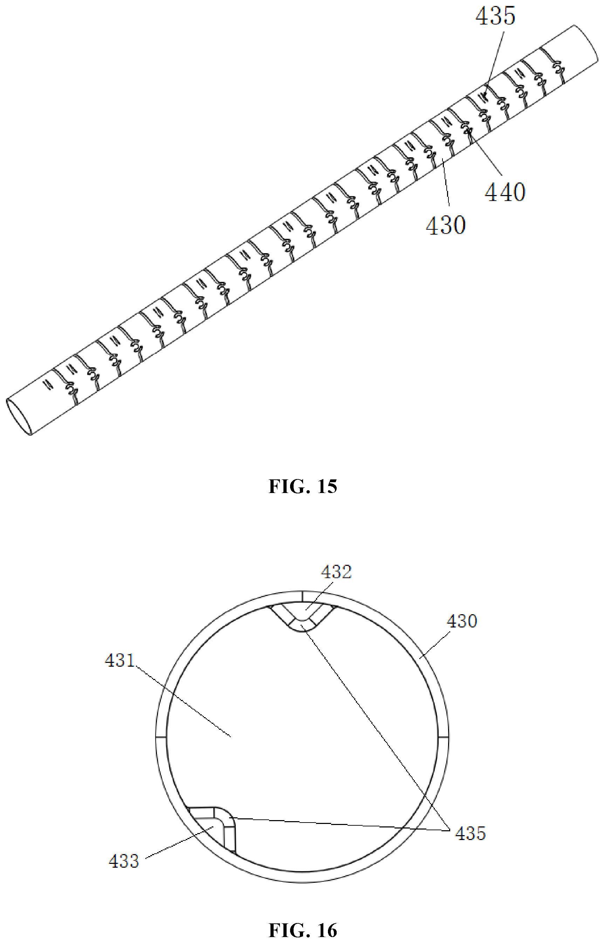

In the above embodiments, the bending segments 430 and the hinge portion 440 are integrally formed by injection molding. In other embodiments, as shown in FIGS. 15 and 16, a metallic bending section is adopted. Specifically, the bending segments 430 of the metallic bending section are configured as an annular structure. A recess 435 is formed by locally stamping and deforming of the annular body, wherein the recess 435 protrudes toward the inner cavity of the annular structure to thereby define a pull-wire channel. To enable pivotal movement between two adjacent bending segments 430 in the metallic bending section, a physical pivot shaft 441 may be formed by machining corresponding grooves and hinge plates at both ends of the metal ring. Alternatively, a riveting structure may be employed between the bending segments 430 to form the physical pivot shaft 441. The formation method of the bending segments 430 in the aforementioned metallic bending section may utilize existing techniques, while only the arrangement positions of the first pull-wire channel 432 and the second pull-wire channel 433 require modification.

During assembly of the insertion tube assembly for the endoscope, the proximal and distal ends of the bending section require connection to respective components. The outer diameter and the wall thickness of the internal channel of these components are limited. By configuring the first pull-wire channel 432 and the second pull-wire channel 433 as an asymmetric structure, at least one is offset along the second direction on the bending segments 430, so that the corresponding components may be more flexibly avoided without increasing the radial size of the insertion tube during assembly to reduce or avoid interference, thereby achieving a reduced radial size of the insertion tube.

In the aforementioned embodiments, the articulation joints are uniformly arranged along the extension axis of the bending section, and each bending segment forms a co-directional bending section with the same deflection direction. The pivot axes of all bending segments are parallel to each other, and consequently, the adjustable articulation section permits reciprocal articulation exclusively along two opposing directions. In alternative configurations, for the adjustable articulation section that needs to be bent in four directions, the two bending segments on both sides of the middle bending segment among the adjacent three bending segments exhibit a 90-degree position difference in the circumferential direction. Relying on the two groups of hinge portions on both sides of the bending segment arranged at 90 degrees in the circumferential direction of the adjustable articulation section, the pivotal motions with crossed pivot axes can be achieved respectively. And, the pull wires are provided with two pairs, and two drive wheels may be set on the bending adjustment drive device each drive one pair of pull wires respectively.

The specific embodiments described herein are presented only to illustrate the principle of the present invention and shall not be construed as limiting the scope of the present invention. For those skilled in the art, various simple deductions, modifications, or substitutions can be made based on the concepts of the present invention.

## Claims

1. An articulation drive mechanism, comprising:
a mounting base;
a drive wheel, rotatably mounted to the mounting base; and
at least one pair of pull wires, two pull wires of each pair arranged on opposite sides of a bending section to drive the bending section to pivot in two opposite directions,
wherein the drive wheel is provided with at least two wire-winding regions comprising at least one first wire-winding region and at least one second wire-winding region;
the first wire-winding region and the second wire-winding region are disposed at radially opposed sides of the drive wheel; and
a winding radius of the at least one first wire-winding region differs from a winding radius of the at least one second wire-winding region.

2. The articulation drive mechanism according to claim 1, wherein:
the at least two wire-winding regions are arranged in layers along a rotational axis of the drive wheel,
grooves are provided in a circumferential surface of the drive wheel, and
each wire-winding region is defined by a respective groove.

3. The articulation drive mechanism according to claim 2, wherein:
the drive wheel comprises an anti-detachment protrusion disposed on a sidewall of the groove and positioned at a rim of the groove, the anti-detachment protrusion being configured to prevent disengagement of the pull wires from the groove in a radial direction of the drive wheel.

4. The articulation drive mechanism according to claim 3, wherein:
the anti-detachment protrusion is disposed on one sidewall of the groove,
a clearance opening is formed on the opposite sidewall of the groove,
at least a portion of the anti-detachment protrusion protrudes into the clearance opening, and
a gap is provided between the anti-detachment protrusion and an inner sidewall of the clearance opening.

5. The articulation drive mechanism according to any one of claims 1 to 4, wherein:
the drive wheel includes an arc segment and a notch portion,
the wire-winding regions are disposed on the arc segment,
at least a portion of an end surface of the arc segment is exposed within the notch portion, and
the pull wires of the articulation drive mechanism are wound onto the arc segment via the notch portion.

6. The articulation drive mechanism according to any one of claims 1 to 4, wherein:
a fixing groove is disposed on an end surface of the drive wheel,
an end of each pull wire is secured to a pull block;
the pull block is received in the fixing groove, and
a wall of the fixing groove is provided with a passage configured to lead the pull wires out.

7. An endoscope, comprising:
an insertion tube assembly, wherein the insertion tube assembly comprises an adjustable articulation section, the adjustable articulation section comprises sequentially hinged bending segments, a hinge portions are disposed between each pair of adjacent bending segments, the hinge portions define a pivot axis configured for relative deflection between the adjacent bending segments, and the hinge portions between each pair of adjacent bending segments are disposed at two radially opposite sides of the adjustable articulation section; and
a handle assembly, wherein the handle assembly is connected to a proximal end of the insertion tube assembly, and the handle assembly incorporates the articulation drive mechanism according to any one of claims 1 to 6.

8. The endoscope according to claim 7, wherein:
an extension direction of an axis of the bending segments is a first direction,
the pivot axis extends in a second direction,
each bending segment has a third direction perpendicular to both the first direction and the second direction,
passages for the pull wires comprise a first pull-wire channel and a second pull-wire channel respectively disposed on two sides of the pivot axis along the third direction, and
at least one of the first pull-wire channel and the second pull-wire channel is offset along the second direction on the bending segments.

9. The endoscope according to claim 8, wherein:
a perpendicular line from the first pull-wire channel to the pivot axis passes through the axis of the bending segments,
the second pull-wire channel is offset, at least in the second direction, relative to a symmetrical position of the first pull-wire channel,
the symmetrical position of the first pull-wire channel is a mirror image position of the first pull-wire channel with respect to a symmetry plane containing both the axis of the bending segments and the pivot axis.

10. The endoscope according to claim 8, wherein:
the bending segment is provided with: one working channel forming a channel distribution region; or at least two working channels, wherein portions of the bending segment between the working channels and the working channels collectively form a channel distribution region;
the first pull-wire channel is disposed externally to the channel distribution region in the third direction, and
the second pull-wire channel is disposed externally to the channel distribution region in the second direction.
